# FASCICULE DE BREVET EUROPEEN

(11) **EP 1 216 722 B1**
(45) Date de publication et mention de la délivrance du brevet: **03.09.2003**
(21) Numéro de dépôt: 01403189.2
(22) Date de dépôt: 11.12.2001
(51) Int. Cl.: A61N 1/37, A61N 1/368

(54) **Dispositif médical actif implantable, notamment stimulateur cardiaque, défibrillateur ou cardioverteur, à détection des situations de fusions**
Aktive implantierbare medizinische Vorrichtung, insbesondere Herzstimulator, Defibrillator und/oder Kardiovertierer mit Mitteln zur Detektion des Zusammenfalls des Reizungpulsklopfens
Active implantable medical device, in particular pacemaker, defibrillator and/or cardioverter having means for detecting fusion

(30) Priorité: 12.12.2000 FR 0016098
(43) Date de publication de la demande: 26.06.2002
(73) Titulaire: ELA MEDICAL, 92541 Montrouge (FR)
(72) Inventeur: Limousin, Marcel, 75014 Paris (FR)
(74) Mandataire: Dupuis-Latour, Dominique

(56) Documents cités:
- WO-A-93/02741
- US-A- 4 969 464
- US-A- 5 626 620
- US-A- 5 855 594

## Description

L'invention concerne les "dispositifs médicaux implantables actifs" tels que définis par la directive 90/385/CEE du 20 juin 1990 du Conseil des communautés européennes, plus précisément les dispositifs stimulateurs cardiaques, défibrillateurs et/ou cardioverteurs permettant de délivrer au coeur des impulsions électriques de faible énergie pour le traitement des troubles du rythme cardiaque.

Elle concerne plus particulièrement l'ajustement de l'amplitude (niveau de tension) des impulsions de stimulation au fil du temps.

Le niveau de stimulation ventriculaire est une valeur typiquement comprise entre 1,5 et 5 V (±10 %), réglable par pas de 0,25 V. Cette amplitude doit être suffisamment élevée pour provoquer la dépolarisation de la cavité myocardique ; il faut cependant éviter des valeurs trop élevées pour ménager la durée de vie de la pile, car l'énergie de stimulation appliquée au myocarde, donc la consommation correspondante du dispositif, est proportionnelle au carré de l'amplitude (et également à la durée) de l'impulsion.

De plus, le seuil est une grandeur qui peut varier dans le temps, de sorte qu'il est souhaitable de pouvoir réévaluer à intervalles réguliers le niveau de l'amplitude de stimulation en opérant un test du seuil d'efficacité de la stimulation, appelé "test de capture". L'amplitude des impulsions de stimulation est ensuite ajustée sur la base du seuil de capture ainsi mesuré, typiquement à un niveau égal au double de la valeur du seuil mesuré, sous réserve d'un niveau minimum (typiquement 1,5 V) et d'un niveau maximum (typiquement 5 V) d'amplitude.

Le WO-A-93/02741 (Ela Médical) décrit ainsi un algorithme de test automatique du seuil de capture ventriculaire, mis en oeuvre dans le stimulateur de type *Talent* commercialisé par Ela Médical.

Un suivi clinique des patients équipés de cet appareil a révélé que dans certains cas l'algorithme de test de seuil automatique voit son efficacité amoindrie en raison d'anomalies survenant au moment du test. Ces anomalies se traduisent par une surévaluation de la valeur du seuil de capture par rapport au seuil réel du patient. Le réajustement de l'amplitude de stimulation étant opéré toutes les six heures, ce niveau excessif est maintenu pendant au moins six heures et, bien qu'il ne soit pas en lui-même dangereux, constitue une source de surconsommation et donc de réduction de la durée de vie de l'implant.

Plus précisément, l'algorithme de détermination automatique du seuil de capture comporte deux phases spécifiques, à savoir :
- une phase préalable de calibration, pour supprimer l'effet de la polarisation de la sonde à l'interface coeur/électrode en déterminant une valeur de référence, puis
- une phase de mesure du seuil d'efficacité, par rapport à la valeur de référence préalablement obtenue.

Comme on le comprend aisément, l'exactitude de l'ajustement est conditionnée par l'exactitude et l'absence d'erreur de la calibration préalable.

Le mécanisme d'une possible erreur de calibration est le suivant.

La phase de calibration comporte au moins deux mesures du potentiel évoqué (c'est-à-dire du potentiel d'une dépolarisation consécutive à une impulsion de stimulation antérieure) pour des impulsions à des amplitudes différentes, par exemple à 2 volts et 4 volts. La calibration est considérée comme efficace si l'amplitude du potentiel évoqué après la stimulation de calibration à 2 volts est supérieure à un certain pourcentage (typiquement 25 %) de celui mesuré à 4 volts.

Mais ce critère peut être vérifié pour une raison autre qu'une capture efficace, ce qui peut survenir dans deux circonstances :
- polarisation très importante de l'interface coeur/électrode et/ou
- fusion, c'est-à-dire stimulation intervenant de façon concomitante à un événement QRS spontané survenant au moment du test de capture.

En effet, le test à 4 volts est toujours efficace (sauf en cas de déplacement de la sonde, mais il s'agit là d'un cas extrême) et l'appareil mesurera donc un potentiel évoqué. En revanche, le test à 2 volts est inefficace (absence de capture), mais le stimulateur mesure néanmoins un potentiel qui, en fait, est soit un potentiel de polarisation élevé, soit un potentiel de dépolarisation résultant d'une fusion, ce qui a pour effet de leurrer le système et entraîner une référence erronée.

Dans la phase subséquente de mesure du seuil d'entraînement, le stimulateur va mesurer systématiquement, pour chaque cycle, la même valeur de potentiel évoqué en dépit de la réduction progressive du niveau de l'amplitude de stimulation. En effet, ce potentiel évoqué mesuré ne sera en fait que le potentiel de polarisation, ou le potentiel de dépolarisation spontanée résultant d'une fusion, par principe toujours supérieurs à 75 % de la valeur de référence, entraînant la conclusion erronée d'une stimulation efficace à chaque cycle.

Le US-A-5 855 594 divulgue une méthode de détermination de la présence d'une tension élevée de polarisation des électrodes. Lors d'un test de vérification de la capture, le stimulateur émet des impulsions à très faible énergie en-dessous du seuil d'entraînement cardiaque. La détection d'un signal lors de telles stimulations est le signe d'une polarisation importante au niveau des électrodes. Toutefois ce brevet n'envisage pas le problème de la fusion.

Le but de l'invention est de proposer un dispositif comportant des moyens pour invalider la calibration et/ou le réajustement du niveau de stimulation en cas de détection de faux positifs sur fusion.

Pour ce faire, l'invention propose un dispositif médical actif implantable, notamment un stimulateur cardiaque, défibrillateur ou cardioverteur, du type générique enseigné par le US-A-5 855 594 précité, c'est-à-dire comprenant des moyens de stimulation, pour délivrer au coeur des impulsions électriques présentant une amplitude et une durée prédéterminées, et des moyens d'ajustement de l'amplitude de ces impulsions de stimulation, comportant des moyens de calibration, des moyens pour délivrer une impulsion de stimulation à tension nulle ou essentiellement nulle, et des moyens de mesure automatique du seuil de capture ventriculaire, comprenant des moyens pour détecter la présence ou l'absence d'une capture consécutive à une stimulation.

Selon l'invention , le dispositif comprend en outre des moyens de détection d'une situation de fusion, aptes à invalider ledit ajustement des impulsions de stimulation en cas de fusion détectée, ces moyens opérant par délivrance d'une stimulation à tension nulle ou essentiellement nulle, et sont aptes à déterminer la présence d'une fusion en cas de détection d'une capture sur une telle stimulation à tension nulle ou essentiellement nulle.

En particulier, lorsque les moyens de détection d'une situation de fusion détectent une capture sur stimulation à tension nulle, ces moyens commandent la réitération de la mise en oeuvre des moyens de calibration, avantageusement avec réduction préalable de l'intervalle d'échappement et/ou du délai atrio-ventriculaire.

En variante ou en complément, les moyens de détection d'une situation de fusion sont des moyens aptes à :
- délivrer une séquence d'impulsions de stimulation à différents niveaux de tension successifs décroissants et à détecter à chaque fois la présence ou la perte de capture, l'amplitude de stimulation étant ajustée en fonction du niveau de l'impulsion ayant entraîné la perte de la capture, et
- après application de la dernière impulsion ayant entraîné une capture corrélative, ou de la dernière impulsion de la séquence, appliquer une impulsion à tension nulle et valident l'ajustement du niveau d'amplitude seulement lorsque l'amplitude du potentiel évoqué sur stimulation à tension nulle est inférieure au potentiel évoqué après ladite dernière stimulation ayant entraîné une capture corrélative.

Dans tous les cas, le dispositif délivre très avantageusement une contre-stimulation après chaque délivrance d'une stimulation à tension nulle.

On va maintenant décrire un exemple de mise en oeuvre de l'invention, en référence aux dessins annexés.
La figure 1 est un graphe donnant le potentiel en fonction de l'amplitude de l'impulsion de stimulation, avec la droite de régression et la référence de mesure du seuil de capture.
La figure 2 est un exemple d'organigramme de mise en oeuvre des enseignements de l'invention.

Les étapes de calibration et de mesure (ou test) sont identiques à celles opérées dans les dispositifs connus, par exemple de la manière décrite dans le WO-A-93/02741 précité, l'invention ne modifiant pas la manière dont chacune de ces deux étapes est en elle-même mise en oeuvre.

Plus précisément, comme on l'a évoqué plus haut, l'algorithme de détermination automatique du seuil de capture comporte deux phases spécifiques, à savoir une phase préalable de calibration qui permet de déterminer une valeur de référence, puis une phase de recherche de seuil, pour mesurer le seuil d'efficacité, mesure opérée par rapport à la valeur de référence préalablement obtenue.

Pour la calibration, le stimulateur mesure le potentiel évoqué (ou, plus précisément, la moyenne de plusieurs valeurs de potentiels évoqués) pour des impulsions d'amplitudes différentes, par exemple à 2 volts et 4 volts.

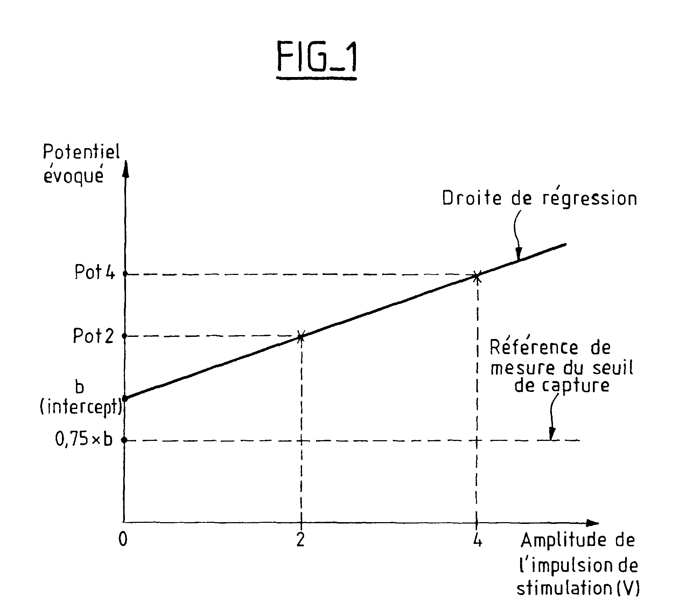

La figure 1 représente les potentiels évoqués Pot2, pour une amplitude de stimulation à 2 volts, et Pot4, pour une amplitude de stimulation à 4 volts. L'algorithme détermine la droite de régression entre ces deux valeurs et l'ordonnée à l'origine, ou intercept, b de cette droite. Le seuil d'efficacité de la capture est fixé à une valeur fonction de cet intercept, par exemple 75 % de la valeur de l'intercept (car l'intercept surestime en fait la valeur réelle de la polarisation), ce qui constitue la valeur de référence par rapport à laquelle sera opérée la mesure du seuil de capture.

L'étape suivante consiste à déterminer le franchissement du seuil de capture de manière à ajuster le niveau de l'amplitude de l'impulsion de stimulation par rapport au "seuil d'entraînement", c'est-à-dire le niveau minimal permettant la capture. Cet ajustement est obtenu par réduction contrôlée progressive du niveau de l'amplitude sur plusieurs cycles successifs, détection de la disparition de la capture, puis rétablissement de l'amplitude à un niveau légèrement supérieur au seuil correspondant à cette disparition.

Le test fournit trois résultats possibles :
1°) un indicateur "ÉCHEC" signifiant que, du fait de résultats aberrants à la calibration, le test ne peut fournir aucune valeur. La valeur de l'amplitude de stimulation est alors fixée automatiquement à la valeur maximale de 5 V pour les six heures suivantes.
2°) un indicateur "RECHERCHE" signifiant que l'algorithme n'a pas pu se dérouler correctement. Les conditions de lancement du test n'étant pas réunies au cours des six heures précédentes (par exemple en raison d'un rythme trop rapide ou d'extrasystoles auriculaires), l'amplitude de stimulation est forcée à la valeur maximale de 5 V en attendant de pouvoir à nouveau lancer le test. Si cette situation perdure six heures de suite, alors l'algorithme produit l'indicateur "RECHERCHE" qui est enregistré dans le fichier de statistiques du stimulateur.
3°) un indicateur "OK" avec une valeur de mesure associée correspondant au dernier seuil de capture efficace trouvé par l'algorithme. Cette valeur est celle qui sera mémorisée par le stimulateur et qui servira à définir l'amplitude de stimulation ventriculaire pour les six prochaines heures.

L'invention est basée sur la constatation que, après une étape de calibration considérée comme effective, malgré que l'algorithme ne puisse pas déterminer avec certitude s'il y a eu ou non capture, il est certain que, si l'on délivre une stimulation avec une amplitude de zéro volt, il n'y aura pas capture.

L'invention propose donc, pour valider ou invalider une calibration, de délivrer une impulsion de stimulation à zéro volt pendant un cycle, et d'analyser l'amplitude du potentiel correspondant évoqué pour valider ou invalider la calibration.

Dans une première mise en oeuvre, après la phase de calibration et avant de commencer la mesure du seuil par délivrance d'impulsions d'amplitudes successives décroissantes, l'algorithme déclenche une stimulation préalable, sur un cycle, avec une amplitude de zéro volt. Si le potentiel évoqué est supérieur à la référence donnée par l'étape de calibration, alors la calibration est considérée comme invalide et il faut soit la recommencer à l'identique, soit la recommencer en assurant la capture par un raccourcissement significatif de l'intervalle d'échappement (IE) et/ou du délai atrio-ventriculaire (DAV), pour éviter d'être en conflit avec la conduction spontanée et empêcher la survenue d'une situation de fusion.

Une autre mise en oeuvre consiste à effectuer la calibration puis le test de seuil de la manière classique, avec des impulsions successives d'amplitudes décroissantes et, après le premier stimulus inefficace (ou après le dernier stimulus de la série, d'amplitude minimale 0,5 volt) de délivrer un stimulus à zéro volt pendant un cycle. L'amplitude du potentiel évoqué après ce dernier stimulus est alors comparée à celle de la dernière impulsion efficace : si la différence n'est pas significative (par exemple moins de 50 %), alors le test est invalidé et la mesure, y compris la calibration, doit être refaite.

Dans tous les cas, par sécurité, une contre-stimulation est délivrée après chaque mesure sur un cycle à amplitude zéro volt.

Les deux manières de procéder que l'on vient d'indiquer peuvent être mises en oeuvre alternativement ou conjointement par l'algorithme, en fonction des circonstances.

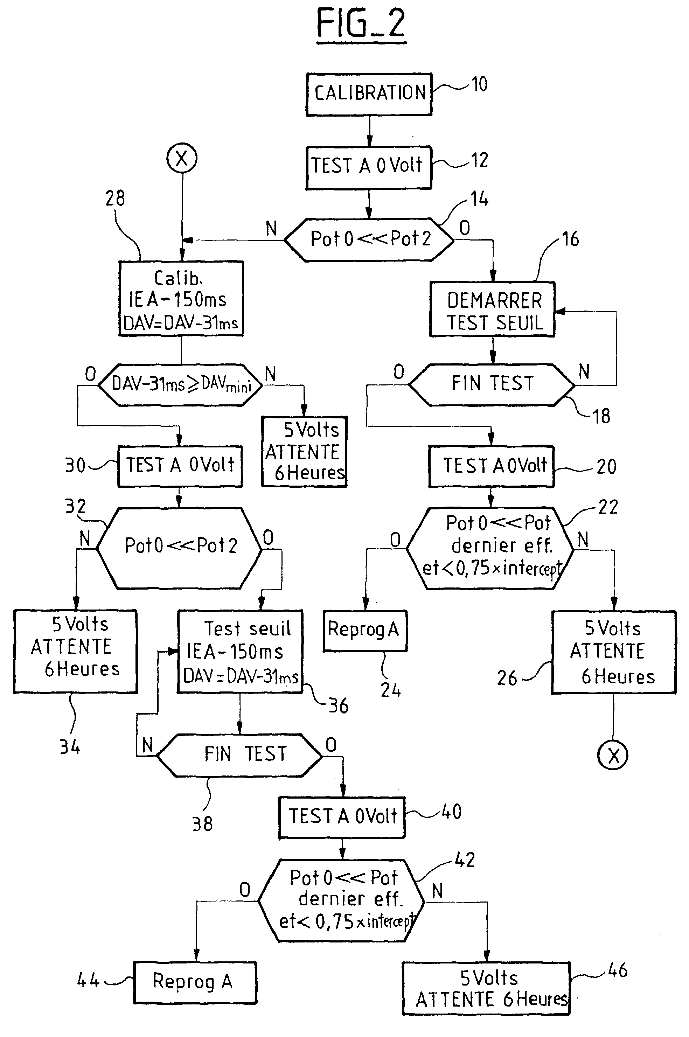

La figure 2 donne un exemple détaillé d'algorithme mettant en oeuvre les enseignements de l'invention.

Après une première calibration initiale (étape 10), un test à zéro volt (étape 12) est effectué, c'est-à-dire qu'une stimulation avec une amplitude de zéro volt est délivrée par le stimulateur.

Le potentiel évoqué Pot0 sur cette stimulation à zéro volt est alors comparé au potentiel évoqué Pot2 à 2 volts lors de la phase de calibration (étape 14).

Si Pot0 est très inférieur à Pot2, la calibration est validée et le test de seuil (étape 16) peut alors commencer, par délivrance d'impulsions successives avec un niveau d'amplitude décroissant sur plusieurs cycles.

Une fois détectée la disparition de la capture (étape 18), un nouveau test à zéro volt est opéré (étape 20).

Si le potentiel évoqué Pot0 correspondant est très inférieur au potentiel évoqué de la dernière impulsion efficace du test de seuil et s'il est inférieur à la valeur de référence (étape 22), alors l'amplitude A du niveau de stimulation est reprogrammée en fonction de la nouvelle valeur du seuil de capture (étape 24) ; dans le cas contraire, l'amplitude de stimulation est forcée, par sécurité, à son niveau maximal de 5 volts et le réajustement du niveau d'amplitude sera reporté à un moment ultérieur, par exemple six heures plus tard (étape 26). Il est également possible de reprendre le test à l'étape 28.

Si, à l'étape 14, Pot0 s'avère non notablement inférieur à Pot2, laissant supposer une situation de fusion, les paramètres de stimulation sont modifiés (étape 28), par exemple l'intervalle d'échappement est diminué de 150 ms et le délai atrio-ventriculaire est diminué, par exemple de 31 ms. Avant d'effectuer cette diminution, l'algorithme s'assure que la valeur DAV-31 ms est supérieure ou égale à la limite minimale fixée dans l'appareil, par exemple 78 ms. S'il n'est pas possible de diminuer de 31 ms le DAV programmé, alors l'algorithme passe directement à l'étape 46.

Un nouveau test à zéro volt est alors effectué (étape 30).

Si le potentiel évoqué Pot0 correspondant n'est pas notablement inférieur à Pot2, ou n'est pas inférieur à la valeur de référence (étape 32), alors l'amplitude de stimulation est forcée, par sécurité, à son niveau maximal de 5 volts et le réajustement du niveau d'amplitude sera reporté à un moment ultérieur, par exemple six heures plus tard (étape 34).

Dans le cas contraire, la calibration est validée et le test de seuil est effectué, avec les paramètres de stimulation modifiés (étape 36). Une fois détectée la disparition de la capture (étape 38), un nouveau test à zéro volt est opéré (étape 40). Si le potentiel évoqué Pot0 correspondant est très inférieur au potentiel évoqué de la dernière impulsion efficace du test de seuil et s'il est inférieur à la valeur de référence (étape 42), alors l'amplitude A du niveau de stimulation est reprogrammée en fonction de la nouvelle valeur du seuil de capture (étape 44) ; dans le cas contraire, l'amplitude de stimulation est forcée, par sécurité, à son niveau maximal de 5 volts et le réajustement du niveau d'amplitude sera reporté à un moment ultérieur, par exemple six heures plus tard (étape 46).

## Revendications

1. Un dispositif médical actif implantable, notamment un stimulateur cardiaque, défibrillateur ou cardioverteur, comprenant :
- des moyens de stimulation, pour délivrer au coeur des impulsions électriques présentant une amplitude et une durée prédéterminées, et
- des moyens d'ajustement de l'amplitude de ces impulsions de stimulation, comportant :
. des moyens de calibration (10 ; 28),
. des moyens pour délivrer une impulsion de stimulation à tension nulle ou essentiellement nulle, et
. des moyens de mesure automatique du seuil de capture ventriculaire, comprenant des moyens pour détecter la présence ou l'absence d'une capture consécutive à une stimulation,
dispositif **caractérisé :**
- **en ce qu'**il comprend en outre des moyens (12, 14, 16 ; 18, 20, 22 ; 30, 32, 36 ; 38, 40, 42) de détection d'une situation de fusion, aptes à invalider ledit ajustement des impulsions de stimulation en cas de fusion détectée, et
- **en ce que** ces moyens de détection opèrent par délivrance d'une stimulation à tension nulle ou essentiellement nulle, et sont aptes à déterminer la présence d'une fusion en cas de détection d'une capture sur une telle stimulation à tension nulle ou essentiellement nulle.

2. Le dispositif de la revendication 1, dans lequel, lorsque les moyens de détection d'une situation de fusion (12, 14 ; 30, 32) détectent une capture sur stimulation à tension nulle, ces moyens commandent la réitération (28) de la mise en oeuvre des moyens de calibration.

3. Le dispositif de la revendication 2, dans lequel la réitération (28) de la mise en oeuvre des moyens de calibration est opérée avec réduction préalable de l'intervalle d'échappement et/ou du délai atrio-ventriculaire.

4. Le dispositif de la revendication 1, dans lequel les moyens de détection d'une situation de fusion sont des moyens aptes à :
- délivrer une séquence d'impulsions de stimulation à différents niveaux de tension successifs décroissants et à détecter à chaque fois la présence ou la perte de capture, l'amplitude de stimulation étant ajustée (24, 44) en fonction du niveau de l'impulsion ayant entraîné la perte de la capture, et
- après application de la dernière impulsion ayant entraîné une capture corrélative, ou de la dernière impulsion de la séquence, appliquer une impulsion à tension nulle et valident l'ajustement du niveau d'amplitude seulement lorsque l'amplitude du potentiel évoqué sur stimulation à tension nulle est inférieure au potentiel évoqué après ladite dernière stimulation ayant entraîné une capture corrélative.

5. Le dispositif de la revendication 1, dans lequel le dispositif délivre une contre-stimulation après chaque délivrance d'une stimulation à tension nulle.

## Patentansprüche

1. Implantierbare, aktive medizinische Vorrichtung, insbesondere Herzschrittmacher, Defibrillator oder Herzwandler, aufweisend:
- Stimulationsmittel, um dem Herzen elektrische Impulse zuzuführen, welche eine vorbestimmte Amplitude und Dauer aufweisen, und
- Mittel zum Einstellen der Amplitude der Stimulationsimpulse, aufweisend:
• Kalibrierungsmittel (10; 28),
• Mittel zum Zuführen eines Stimulationsimpulses mit Spannung null oder im Wesentlichen null, und
• Mittel zum automatischen Messen der Schwelle der ventrikulären Schließung, Mittel aufweisend zum Detektieren der Anwesenheit oder der Abwesenheit einer Schließung in der Folge einer Stimulation,
wobei die Vorrichtung **gekennzeichnet ist:**
- **dadurch**, dass sie des Weiteren Mittel (12, 14, 16; 18, 20, 22; 30, 32, 36; 38, 40, 42) aufweist zum Detektieren einer Fusionssituation, die geeignet sind, die Einstellung der Stimulationsimpulse aufzuheben im Fall der detektierten Fusion, und
- **dadurch**, dass die Detektionsmittel arbeiten durch Abgabe einer Stimulation mit der Spannung null oder im Wesentlichen null, und geeignet sind, die Anwesenheit einer Fusion zu bestimmen im Fall der Detektion einer Schließung auf eine derartige Stimulation mit Spannung null oder im Wesentlichen null.

2. Vorrichtung gemäß Anspruch 1, bei welcher, wenn die Detektionsmittel für eine Fusionssituation (12, 14; 30, 32) eine Schließung auf Stimulation mit Spannung null detektieren, diese Mittel den Wiederdurchlauf (28) der Inbetriebnahme der Kalibrierungsmittel befehlen.

3. Vorrichtung gemäß Anspruch 2, bei welcher der Wiederdurchlauf (28) der Inbetriebnahme der Kalibrierungsmittel durchgeführt wird mit vorheriger Reduzierung des Auslassintervalls und/oder der atrio-ventrikulären Verzögerung.

4. Vorrichtung gemäß Anspruch 1, bei welcher die Detektionsmittel einer Fusionssituation Mittel sind, die geeignet sind zum:
- Abgeben einer Stimulationsimpulssequenz mit abnehmenden nachfolgenden verschiedenen Spannungsniveaus und zum Detektieren jedes Mal die Anwesenheit oder den Verlust der Schließung, wobei die Stimulationsamplitude eingestellt wird (24, 44) in Abhängigkeit des Niveaus des Impulses, der den Verlust der Schließung nach sich gezogen hat, und
- nach Zuführung des letzten Impulses, der eine korrelative Schließung nach sich gezogen hat, oder des letzten Impulses der Sequenz, Zuführen eines Impulses der Spannung null und Validieren der Einstellung des Amplitudenniveaus nur, wenn die Amplitude des Potentials, welches hervorgerufen wird auf Stimulation mit Spannung null, unterhalb des Potentials ist, welches hervorgerufen worden ist nach der letzten Stimulation, welche eine korrelative Schließung nach sich gezogen hat.

5. Vorrichtung gemäß Anspruch 1, bei welcher die Vorrichtung eine Gegenstimulation abgibt nach jeder Abgabe einer Stimulation mit Spannung null.

## Claims

1. An active implantable medical device, in particular a pacemaker, defibrillator or cardioverter, including:
- stimulation means, for delivering to-the heart electrical pulses presenting a predetermined amplitude and duration, and
- means for adjusting the amplitude of said stimulation pulses, comprising:
· calibrating means (10 ; 28),
· means for delivering a stimulation pulse at null or essentially null voltage, and
· means for automatically measuring the ventricular capture threshold, including means for detecting a presence or an absence of a capture consecutive to a stimulation,
said device being **characterized:**
- **in that** it further includes means (12, 14, 16 ; 18, 20, 22 ; 30, 32, 36 ; 38, 40, 42) for detecting a fusion situation, adapted to invalidate said adjustment of the stimulation pulses in the event of a detected fusion, and
- **in that** said means operate by delivering a stimulation pulse at null or essentially null voltage, and are adapted to determine the presence of a fusion in the event of detection of a capture in response to such a stimulation at null or essentially null voltage.

2. The device of claim 1, wherein, when the means for detecting a fusion situation (12, 14 ; 30, 32) detect a capture in response to a stimulation at null voltage, said means control the reiteration (28) of the operation of the calibrating means.

3. The device of claim 2, wherein the reiteration (28) of the operation of the calibrating means is done with a preliminary reduction of the escape interval and/or the atrioventricular delay.

4. The device of claim 1, wherein the means for detecting a fusion situation are means adapted to:
- deliver a sequence of stimulation pulses at various decreasing successive voltage levels and to detect each time the presence or the loss of capture, the stimulation amplitude being adjusted (24, 44) according to the level of the pulse that produced the loss of the capture, and
- after application of the last pulse producing a correlative capture, or of the last pulse of the sequence, apply a pulse at null voltage and validate the adjustment of the threshold amplitude level only when the amplitude of the evoked potential for a stimulation at null voltage is lower than the evoked potential after said last stimulation producing a correlative capture.

5. The device of claim 1, wherein the device delivers a counter-stimulation after each delivery of a stimulation at null voltage.
